# EUROPEAN PATENT APPLICATION

(11) **EP 3 006 088 A1**
(43) Date of publication of application: **13.04.2016**
(21) Application number: 15187170.4
(22) Date of filing: 28.09.2015
(51) Int. Cl.: A61Q 19/10, A61K 8/41, A61K 8/60

(54) **CLEANSING COMPOSITIONS**

(30) Priority: 29.09.2014 US 201462056759 P
(71) Applicant: Johnson & Johnson Consumer Inc., Skillman, NJ 08558 (US)
(72) Inventor: NOGUEIRA,, Ana Carolina Santos, Skillman, NJ New Jersey 08558 (US); RINALDI,, Raphael H., 12240907 Sao Paulo (BR)
(74) Representative: Carpmaels & Ransford LLP

(57) **Abstract**

The present invention relates to cleansing compositions including: (a) from about 3% to about 20% by weight based on the total weight of the composition amphoteric surfactant; and (b) an alkyl glucoside surfactant, wherein a ratio of alkyl glucoside surfactant to amphoteric surfactant is at least 1 to 1, the composition is free of ethoxylated surfactants, and the composition has a viscosity of at least 1000 centipoise.

## Description

### Cross-Reference to related Application

This application claims the benefit of priority to U.S. Provisional Application Serial No. 62/056759 filed September 29, 2014.

### Field of the Invention

The present invention relates to cleansing compositions that are useful in cleansing the skin and hair and are characterized by having low irritation characteristics in combination with sufficient viscosity in the absence of ethoxylated surfactants.

### Background of the Invention

Cleansing compositions typically contain nonionic, anionic and/or amphoteric surfactants. Of the nonionic surfactants, ethoxylated surfactants are typically utilized to increase the viscosity of the composition. This leads to less dripping of the product and nice product aesthetics.

However, as is recognized in the art, there is a concern of ethoxylated surfactants potentially containing 1,4-dioxane and associated safety concerns. Therefore, it is desirable to provide cleansing compositions that are free of ethoxylated surfactants.

Removal of ethoxylated surfactants results in low viscosity, which is undesirable. Therefore, there is a need for mild cleansing compositions that are free of ethoxylated surfactants and have sufficient viscosity.

### SUMMARY OF THE INVENTION

The present invention relates to a cleansing composition comprising, consisting essentially of and consisting of: (a) from about 3% to about 20% by weight based on the total weight of the composition of an amphoteric surfactant; and (b) an alkyl glucoside surfactant, wherein a ratio of alkyl glucoside surfactant to amphoteric surfactant is at least 1 to 1, the composition is free of ethoxylated surfactants, and the composition has a viscosity of at least 2000 centipoise.

### DETAILED DESCRIPTION OF THE INVENTION

Applicants have discovered that the skin cleansing compositions of this invention exhibit a unique and unexpected combination of properties including relatively low irritation and relatively high viscosity for non-ethoxylated cleansing compositions. This makes the compositions of this invention ideal for skin and hair care, including baby and infant skin, cosmetic or cleansing compositions.

The term "free of ethoxylated surfactants" means the compositions of the present invention contain less than 1%, for example less than 0.1% or 0% by weight ethoxylated surfactant.

To provide a more concise description, some of the quantitative expressions given herein are not qualified with the term "about". It is understood that whether the term "about" is used explicitly or not, every quantity given herein is meant to refer to the actual given value, and it is also meant to refer to the approximation to such given value that would reasonably be inferred based on the ordinary skill in the art, including approximations due to the experimental and/or measurement conditions for such given value.

To provide a more concise description, some of the quantitative expressions herein are recited as a range from about amount X to about amount Y. It is understood that wherein a range is recited, the range is not limited to the recited upper and lower bounds, but rather includes the full range from about amount X through about amount Y, or any amount or range therein.

### Amphoteric Surfactants

Compositions of the present invention include an amphoteric surfactant. Nonlimiting examples of amphoteric surfactants are those selected from the group consisting of betaines, alkyliminoacetates, iminodialkanoates, and aminoalkanoates.

Examples of amphoteric surfactants of the present invention include disodium lauroamphodiacetate, sodium lauroamphoacetate, cetyl dimethyl betaine, cocoamidopropyl betaine, and oleyl betaine.

Other examples of suitable betaine compounds include dodecyldimethylammonium acetate, tetradecyldimethylammonium acetate, hexadecyldimethylammonium acetate, alkyldimethylammonium acetate wherein the alkyl group averages about 12 to 18 carbon atoms in length, dodecyldimethylammonium butanoate, tetradecyldimethylammonium butanoate, hexadecyldimethylammonium butanoate, dodecyldimethylammonium hexanoate, hexadecyldimethylammonium hexanoate, tetradecyldimethylammonium pentanoate and tetradecyldipropyl ammonium pentanoate. The amount of amphoteric surfactant in the compositions of the present invention may range from about 3% to about 20% by weight, for example from about, 4% to about 18% by weight and from about7% to about 15% by weight based on the total weight of the composition.

### Alkyl Glucoside

Compositions according to the present invention also include an alkyl glucoside surfactant. Suitable alkyl glucoside surfactants include, but are not limited to, decyl glucoside, coco glucoside, lauryl glucoside, and polyglyceryl esters, such as but not limited to polyglyceryl-10 laurate and polyglyceryl-10 oleate. The amount of alkyl glucoside surfactant in the compositions of the present invention may range from about 3% to about 20% by weight, for example from about 4% to about 18% by weight, and from about 7% to about 15% by weight based on the total weight of the composition.

Compositions according to the present invention may also include an anionic surfactant. Suitable anionic surfactants include, but are not limited to, sodium coco sulfate. The amount of anionic surfactant in the compositions of the present invention may range from about 0.1% to about 5% by weight, for example from about 0.1 % to about 3% by weight based on the total weight of the composition. When an anionic surfactant is present in the compositions of the present invention, the ratio of the total amount of alkyl glucoside surfactant and anionic surfactant to amphoteric surfactant is at least 1 to 1.

Compositions according to the present invention have a viscosity of 2000 centipoise or more (Brookfield LVF, 6 rpm, spindle no. 2) at 25°C in a stripped down formulation (water and surfactants). The viscosity of the final formulation is at least 1000 centipoise (under the same testing conditions).

The cleansing compositions produced may further contain any of a variety of other components nonexclusively including additives which enhance the appearance, feel and fragrance of the compositions, such as colorants, fragrances, preservatives, pH adjusting agents and the like.

Any of a variety of non-ethoxylated nonionic surfactants are suitable for use in the compositions of this invention. Examples of suitable nonionic surfactants include, but are not limited to, alkyl polyglucosides, polyglyceryl esters, mixtures thereof, and the like. Certain preferred nonionic surfactants include alkyl polyglucosides, such as but not limited to coco-glucoside and decyl-glucoside, and polyglyceryl esters, such as but not limited to polyglyceryl-10 laurate and polyglyceryl-10 oleate.

Any of a variety of commercially available secondary conditioners, such as volatile silicones, which impart additional attributes, such as gloss to the hair are suitable for use in this invention. In one embodiment, the volatile silicone conditioning agent has an atmospheric pressure boiling point less than about 220°C. The volatile silicone conditioner may be present in an amount of from about 0 percent to about 3 percent, e.g. from about 0.25 percent to about 2.5 percent or from about 0.5 percent to about 1 percent, based on the overall weight of the composition. Examples of suitable volatile silicones nonexclusively include polydimethylsiloxane, polydimethylcyclosiloxane, hexamethyldisiloxane, cyclomethicone fluids such as polydimethylcyclosiloxane available commercially from Dow Corning Corporation of Midland, Michigan under the tradename, "DC-345" and mixtures thereof, and preferably include cyclomethicone fluids.

Any of a variety of commercially available humectants, which are capable of providing moisturization and conditioning properties to the personal cleansing composition, are suitable for use in this invention. The humectant may be present in an amount of from about 0 percent to about 10 percent, e.g. from about 0.5 percent to about 5 percent or from about 0.5 percent to about 3 percent, based on the overall weight of the composition. Examples of suitable humectants nonexclusively include: 1) water soluble liquid polyols selected from the group comprising glycerine, propylene glycol, hexylene glycol, butylene glycol, dipropylene glycol, and mixtures thereof; 2)polyalkylene glycol of the formula: HO-(R"O)_{b}-H, wherein R" is an alkylene group having from about 2 to about 3 carbon atoms and b is an integer of from about 2 to about 10; 3) polyethylene glycol ether of methyl glucose of formula CH₃-C₆H₁₀O₅-(OCH₂CH₂)_{c}-OH, wherein c is an integer from about 5 to about 25; 4) urea; and 5) mixtures thereof, with glycerine being the preferred humectant.

Examples of suitable chelating agents include those which are capable of protecting and preserving the compositions of this invention. Preferably, the chelating agent is ethylenediamine tetracetic acid ("EDTA"), and more preferably is tetrasodium EDTA, available commercially from Dow Chemical Company of Midland, Michigan under the tradename, "Versene 100XL" and is present in an amount, based upon the total weight of the composition, from about 0 to about 0.5 percent or from about 0.05 percent to about 0.25 percent.

Suitable preservatives include organic acid preservatives may include benzoic acid and alkali metal and ammonium salts thereof (e.g. sodium benzoate), sorbic acid and alkali metal and ammonium salts thereof (e.g. potassium sorbate), *p*-Anisic acid and alkali metal and ammonium salts thereof, and salicylic acid and alkali metal and ammonium salts thereof. The pH of the composition may be adjusted to the appropriate acidic value using any cosmetically acceptable organic or inorganic acid, such as citric acid, acetic acid, glycolic acid, lactic acid, malic acid, tartaric acid, or hydrochloric acid.

In one embodiment of the composition, sodium benzoate is present in the composition in an amount, based upon the total weight of the composition, from about 0 to about 0.5 percent. In another embodiment, potassium sorbate is present in the composition in an amount, based upon the total weight of the composition, from about 0 to about 0.6 percent, more preferably from about 0.3 to about 0.5 percent.

The ethoxylate free surfactant cleansing composition according to the present invention may include shampoos, 2 in 1 shampoo-conditioners, hair and body washes, washes, baths, gels, lotions, creams and the like.

The present invention provides for methods of cleansing a portion of the body including the skin and hair comprising contacting the body with the cleansing composition of the present invention.

The methods and compositions of this invention illustratively disclosed herein suitably may be practiced in the absence of any component, ingredient, or step which is not specifically disclosed herein. Several examples are set forth below to further illustrate the nature of the invention and the manner of carrying it out. However, the invention should not be considered as being limited to the details thereof.

### Examples

Surfactant solutions in water were utilized to identify suitable surfactant compositions that are free of ethoxylated surfactants and have sufficient viscosity. The samples in Table 1 below were prepared by the following procedure. The ingredients were added to water (completed to 100% w/w). Decyl-Glucoside was added and mixed for 5 minutes, amphoteric surfactant was added and mixed for 5 minutes, and Sodium Coco Sulfate was added and mixed until solubilized. The pH was adjusted from 5.0 to 6.0 with citric acid.

Viscosities were measured using a Brookfielkd LVF instrument with a number 2 spindle at 6 RPM at 25°C. A viscosity of 2000 or greater was considered acceptable. The results are reported in Table 1 below.

**Table 1**

| Sample | Viscosity (cP) | CAB: DG | CAB (wt%) | DG (wt%) | SCS (wt %) |
|---|---|---|---|---|---|
| 1 | 589.9 | 1.0 : 0.47 | 4.25 | 2 | 1.35 |
| 2 | 1600 | 1.0 : 0.83 | 4.22 | 3.5 | 1.35 |
| 3 | 2230 | 1.0 : 1.08 | 3.00 | 3.25 | 1.35 |
| 4 | 3150 | 1.0 : 1.41 | 3.01 | 4.25 | 1.35 |
| 5 | 2200 | 1.0 : 1.06 | 3.30 | 3.5 | 1.35 |

| | | | | | |
|---|---|---|---|---|---|
| CAB = cocoamidopropylbetaine, DG = decyl glucoside SCS = sodium coco sulfate | | | | | |

The data above shows that in an aqueous formulation, formulas with 3% or more cocoamidopropyl betaine and a ratio of 1 to 1 or greater (alkyl glucoside to amphoteric surfactant) was necessary to achieve acceptable viscosity for the cleansing compositions of the invention. Several formulations were prepared using the ingredients below. The formulas are made by adding a portion (approximately 20% of the water to a first container. To this container gums, if used, are added with slow mixing. In a second container, a second portion of the water (approximately 10%) is added. To this container, p-ansinic acid, if used, is added with sodium hydroxide. In a third container, the remaining water is added. Under mixing, the surfactants are added and heat applied. The first two mixtures, if used, are added to the larger portion. Mixing continues and the fragrances, colors and preservatives are added. The pH is adjusted to 5-6 with citric acid and sodium hydroxide.
Formula 1

| **Trade Name** | **US INCI Name** | **% wt** |
|---|---|---|
| Plantacare 818 UP | Coco-Glucoside | 10.000 |
| Mackol CAS 100N | Sodium Coco-Sulfate | 2.600 |
| Mackam C-37 HP | Cocoamidopropyl Betaine | 10.800 |
| Jaguar C 162 | Hydroxypropyl Guar Hydroxypropyltrimonium Chloride | 0.150 |
| Lamesoft PO65 | Coco-Glucoside; Glyceryl Oleate | 1.000 |
| Dehyquart CC7 BZ | Polyquaternium-7 | 0.500 |
| CUTINA AGS | Glycol Distearate | 0.500 |
| Dissolvine GL-47-S | Tetrasodium Glutamate Diacetate | 0.200 |
| Phenoxyethanol | Phenoxyethanol | 0.600 |
| Sodium Benzoate EMPROVE | Sodium Benzoate | 0.500 |
| Sensiva SC-50 (Schulke & Mayr) | Ethylhexylglycerin | 0.010 |
| Fragrance | Fragrance | 0.270 |
| Citric Acid | Citric Acid | 0.400 |
| Additive Ingredients * | | 0.050 |
| Purified Water | Water | 72.420 |
| | Total | 100.000 |

| | | |
|---|---|---|
| * Oils, proteins and extracts, such as Silk Amino acids, Hydrolyzed Rice Protein, Hydrolyzed Keratin, Aloe Barbadensis Leaf Extract, Hydrolyzed Wheat Protein, Chamomila Recutita Flower Extract, Passiflora Incarnata Seed Oil, Argania Spinosa Kernel Oil and Theobroma Grandiflorum Seed Butter PLANTACARE 818 UP = 50% coco-glucoside. (5% final) MACKAM C-37 HP is 30% cocoamidopropyl betaine. (3.24% final) Cocoamidopropyl betaine to alkyl glucoside ratio = 1 : 1.5 | | |

The viscosity of the final formulation was measured using a Brookfield LVF instrument with a number 2 spindle at 6 RPM at 25°C. The viscosity was 2000 centipoise.
Formula 2

| **Trade Name** | **EUINCIName** | **% wt** |
|---|---|---|
| Plantacare 818 UP | Coco-Glucoside | 10.000 |
| Mackol CAS 100N | Sodium Coco-Sulfate | 2.600 |
| Oxitaine CP 30 CM | Cocamidopropyl Betaine | 10.800 |
| Jaguar C 162 | Hydroxypropyl Guar Hydroxypropyltrimonium Chloride | 0.150 |
| Lamesoft PO65 | Coco-Glucoside; Glyceryl Oleate | 1.000 |
| Dehyquart CC7 BZ | Polyquaternium-7 | 0.500 |
| CUTINA AGS | Glycol Distearate | 0.500 |
| Dissolvine GL-47-S | Tetrasodium Glutamate Diacetate | 0.200 |
| Phenoxyethanol | Phenoxyethanol | 0.600 |
| Sodium Benzoate EMPROVE | Sodium Benzoate | 0.500 |
| Sensiva SC-50 (Schulke & Mayr) | Ethylhexylglycerin | 0.010 |
| Fragrance | Parfum | 0.270 |
| Citric Acid | Citric Acid | 0.400 |
| Additive Ingredients * | | 0.050 |
| Purified Water | Water | 72.420 |
| | **Total:** | 100 |

| | | |
|---|---|---|
| * Oils, proteins and extracts, such as Silk Amino acids, Hydrolyzed Rice Protein, Hydrolyzed Keratin, Aloe Barbadensis Leaf Extract, Hydrolyzed Wheat Protein, Chamomila Recutita Flower Extract, Passiflora Incarnata Seed Oil, Argania Spinosa Kernel Oil and Theobroma Grandiflorum Seed Butter PLANTACARE 818 UP = 50% coco-glucoside. (5% final) OXITAINE CP 30 CM is 30% cocoamidopropyl betaine. (3.24% final) Cocoamidopropyl betaine to alkyl glucoside ratio = 1 : 1.5 | | |

The viscosity of the final formulation was measured using a Brookfield LVF instrument with a number 2 spindle at 6 RPM at 25°C. The viscosity was 2600 centipoise.
Formula 3

| **Trade Name** | **EUINCIName** | | **%wt** |
|---|---|---|---|
| Plantacare 818 UP | Coco-Glucoside | | 10.000 |
| Mackol CAS 100N | Sodium Coco-Sulfate | | 2.600 |
| Amphosol HCA-HP | Cocamidopropyl Betaine | | 10.800 |
| Jaguar C 162 | Hydroxypropyl Guar Hydroxypropyltrimonium Chloride | | 0.150 |
| Lamesoft PO65 | Coco-Glucoside; Glyceryl Oleate | | 1.000 |
| Dehyquart CC7 BZ | Polyquaternium-7 | | 0.500 |
| CUTINA AGS | Glycol Distearate | | 0.500 |
| Dissolvine GL-47-S | Tetrasodium Glutamate Diacetate | | 0.200 |
| PHENOXIETANOL | Phenoxyethanol | | 0.600 |
| SODIUM BENZOATE | Sodium Benzoate | | 0.500 |
| Sensiva SC-50 | Ethylhexylglycerin; Tocopherol | | 0.010 |
| Fragrance | Parfum | | 0.270 |
| Citric Acid | Citric Acid | | 0.400 |
| Additive Ingredients * | Additive Ingredients * | | 0.050 |
| Purified Water | Aqua | | 72.420 |
| | | **Total:** | 100 |

| | | | |
|---|---|---|---|
| * Oils, proteins and extracts, such as Silk Amino acids, Hydrolyzed Rice Protein, Hydrolyzed Keratin, Aloe Barbadensis Leaf Extract, Hydrolyzed Wheat Protein, Chamomila Recutita Flower Extract, Passiflora Incarnata Seed Oil, Argania Spinosa Kernel Oil and Theobroma Grandiflorum Seed Butter PLANTACARE 818 UP = 50% coco-glucoside. (5% final) AMPHOSOL HCA-HP is 30% cocoamidopropyl betaine. (3.24% final) Cocoamidopropyl betaine to alkyl glucoside ratio = 1 : 1.5 | | | |

The viscosity of the final formulation was measured using a Brookfield LVF instrument with a number 2 spindle at 6 RPM at 25°C. The viscosity was 5000 centipoise.
Formula 4

| **Trade Name** | **EU INCI Name** | **% wt** |
|---|---|---|
| Plantacare 818 UP | Coco-Glucoside | 10.000 |
| GALAXY 789SP (Sodium Coco Sulfate) | SODIUM C12-18 ALKYL SULFATE | 2.500 |
| Dehyton PK 45 (Pomezia) | Aqua; Cocamidopropyl Betaine; Sodium Chloride | 8.100 |
| Merquat 7SPR | Water;Polyquatemium-7;Sodium Benzoate | 0.500 |
| Jaguar C 162 | Hydroxypropyl Guar Hydroxypropyltrimonium Chloride | 0.100 |
| Lamesoft PO65 | Coco-Glucoside; Glyceryl Oleate; Aqua; Citric Acid; Hydrogenated Palm Glycerides Citrate; Tocopherol | 1.000 |
| Dissolvine GL-PD-S | Tetrasodium Glutamate Diacetate; Sodium Hydroxide; Aqua | 0.120 |
| Water, Purified (RMT0138) | Aqua | 66.050 |
| Phenoxetanol | Phenoxyethanol | 0.360 |
| Sensiva SC-50 | Ethylhexylglycerin; Tocopherol | 0.070 |
| Dermosoft MM688 RM - Milled from China Zhangjiagang | p-Anisic Acid | 0.200 |
| Sodium Benzoate | Sodium Benzoate | 0.300 |
| Water, Purified (RMT0138) | Aqua | 10.000 |
| Fragrance | Parfum | 0.150 |
| Citric Acid Monohydrate fine granular | Citric Acid | 0.400 |
| Additive Ingredients * | | 0.050 |
| Sodium Hydroxide 100% | Sodium Hydroxide | 0.100 |
| | Total: | 100 |

| | | |
|---|---|---|
| * Oils, proteins and extracts, such as Silk Amino acids, Hydrolyzed Rice Protein, Hydrolyzed Keratin, Aloe Barbadensis Leaf Extract, Hydrolyzed Wheat Protein, Chamomila Recutita Flower Extract, Passiflora Incarnata Seed Oil, Argania Spinosa Kernel Oil and Theobroma Grandiflorum Seed Butter PLANTACARE 818 UP = 50% coco-glucoside. (5% final) DEHYTON PK is 42 to 45% cocoamidopropyl betaine. (3.4 to 3.6% final) Cocoamidopropyl betaine to alkyl glucoside ratio = 1 : 1.39 to 1.47 | | |

The viscosity of the final formulation was measured using a Brookfield LVF instrument with a number 2 spindle at 6 RPM at 25°C. The viscosity was 2200 centipoise.
Formula 5

| **Trade Name** | **US INCI Name** | **%wt** |
|---|---|---|
| Purified Water | Water | 74.500 |
| Jaguar C-162 (Rhodia) | Guar Hydroxypropyltrimonium Chloride | 0.200 |
| Citric Acid Anhydrous Fine Granular 51N USP (DSM Nutritional Products) | Citric Acid | 0.040 |
| Merquat 7SPR (Nalco/Lubrizol) | Polyquaternium-7 | 1.000 |
| Lamesoft PO 65 (BASF) | Coco-Glucoside; Glyceryl Oleate | 1.000 |
| Plantacare 2000 UP (BASF/Cognis) | Decyl Glucoside | 10.000 |
| Tego Betaine F50J Special (Evonik Goldschmidt) | Cocamidopropyl Betaine | 8.700 |
| Euxyl PE 9010 (Schulke & Mayr) | Phenoxyethanol; Ethylhexylglycerin | 0.400 |
| Edeta B Powder (BASF) | Tetrasodium EDTA | 0.100 |
| XINKANG-SB NF (WUHAN YOUJI) | Sodium Benzoate | 0.300 |
| Fragrance | Fragrance | 0.600 |
| D&C Yellow No 10 K7059-C (Sensient) | Yellow 10 | 0.000027 |
| GIVOBIO G Cu (Givaudan-Lavirotte/Isaltis) | Copper Gluconate | 0.200 |
| Mackol CAS-100N (Rhodia Inc) | Sodium Coco-Sulfate | 2.800 |
| Citric Acid) | Citric Acid | 0.150 |
| | **Total** | 100 |

| | | |
|---|---|---|
| Plantacare 2000 N UP = 50% decyl-glucoside. (5.0% final) TEGO BETAINE F50J Special is 50% cocoamidopropyl betaine. (4.35% final) Cocoamidopropyl betaine to alkyl glucoside ratio = 1 : 1.15 | | |

The viscosity of the final formulation was measured using a Brookfield LVF instrument with a number 2 spindle at 6 RPM at 25°C. The viscosity was 2200 centipoise.
Formula 6

| **Trade Name** | **INCI name** | **% (w/w)** |
|---|---|---|
| Plantaren 2000 N UP | Decyl - Glucoside | 10.000 |
| Mackol CAS 100N | Sodium Coco-Sulfate | 1.600 |
| Oxitaine CP 30 | Cocamidopropyl Betaine | 10.800 |
| Merquat 3940 | Polyquaternium 39 | 0.250 |
| Dehyquart CC7 BZ | Polyquaternium 7 | 1.250 |
| CUTINA AGS | Glycol Distearate | 0.700 |
| Dye | Red 33 | 0.000040 |
| Fragrance | Parfum | 0.300 |
| Dissolvine GL-47-S | Tetrasodium Glutamate Diacetate | 0.200 |
| PHENOXIETANOL | Phenoxyethanol | 0.360 |
| Elestab CPN | Chlorfenesin | 0.200 |
| Citric Acid | Citric Acid | 0.400 |
| Additive Ingredients * | | 0.110 |
| Purified Water | Aqua | 73.830 |
| | **TOTAL** | Q.S. - 100 |

| | | |
|---|---|---|
| *Oils, proteins and extracts, such as Silk Amino acids, Hydrolyzed Rice Protein, Hydrolyzed Keratin, Aloe Barbadensis Leaf Extract, Hydrolyzed Wheat Protein, Chamomila Recutita Flower Extract, Passiflora Incarnata Seed Oil, Argania Spinosa Kernel Oil and Theobroma Grandiflorum Seed Butter Plantaren 2000 N UP = 50% decyl-glucoside. (5.0% final) OXITAINE CP 30 CM is 30% cocoamidopropyl betaine. (3.24% final) Cocoamidopropyl betaine to alkyl glucoside ratio = 1 : 1.54 The viscosity of the final formulation was measured using a Brookfield LVF instrument with a number 2 spindle at 6 RPM at 25°C. The viscosity was 1505 centipoise. | | |

Formula 7

| **Trade Name** | **INCI name** | **% (w/w)** |
|---|---|---|
| Plantaren 2000 N UP | Decyl Glucoside | 10.000 |
| Mackol CAS 100N | Sodium Coco-Sulfate | 2.200 |
| Oxitaine CP 30 | Cocamidopropyl Betaine | 10.800 |
| Jaguar C 162 | Hydroxypropyl Guar Hydroxypropyltrimonium Chloride | 0.200 |
| Lamesoft PO65 | Coco-Glucoside; Glyceryl Oleate | 1.000 |
| Dye | Cl 17200 | 0.00005 |
| Dye | Cl 4209 | 0.000225 |
| Dissolvine GL-47-S | Tetrasodium Glutamate Diacetate | 0.200 |
| PHENOXIETANOL | Phenoxyethanol | 0.360 |
| SODIUM BENZOATE | Sodium Benzoate | 0.500 |
| Dermosoft 688 | p-anisic acid | 0.200 |
| Sensiva SC-50 | Ethylhexylglycerin; Tocopherol | 0.150 |
| Dermosoft 688 | p-anisic acid | 0.150 |
| Fragrance | Parfum | 0.300 |
| Citric Acid | Citric Acid | 0.400 |
| Purified Water | Aqua | 73.54 |
| | **TOTAL** | Q.S. - 100 |

| | | |
|---|---|---|
| PLANTAREN 2000 N UP = 50% decyl-glucoside. (5.0% final) OXITAINE CP 30 CM is 30% cocoamidopropyl betaine. (3.24% final) Cocoamidopropyl betaine to alkyl glucoside ratio = 1 : 1.54 | | |

The viscosity of the final formulation was measured using a Brookfield LVF instrument with a number 2 spindle at 6 RPM at 25°C. The viscosity was 1725 centipoise.
Formula 8

| **Trade Name** | **INCI name** | **% (w/w)** |
|---|---|---|
| Plantaren 2000 N UP | Decyl Glucoside | 9.000 |
| Mackol CAS 100N | Sodium Coco-Sulfate | 1.800 |
| Oxitaine CP 30 | Cocamidopropyl Betaine | 9.500 |
| Jaguar C 162 | Hydroxypropyl Guar Hydroxypropyltrimonium Chloride | 0.200 |
| Lamesoft PO65 | Coco-Glucoside; Glyceryl Oleate | 1.000 |
| CUTINA AGS | Glycol Distearate | 0.700 |
| Dissolvine GL-47-S | Tetrasodium Glutamate Diacetate | 0.200 |
| PHENOXIETANOL | Phenoxyethanol | 0.360 |
| SODIUM BENZOATE | Sodium Benzoate | 0.500 |
| Dermosoft 688 | p-anisic acid | 0.200 |
| Sensiva SC-50 | Ethylhexylglycerin; Tocopherol | 0.150 |
| Fragrance | Parfum | 0.300 |
| Citric Acid | Citric Acid | 0.400 |
| Additive Ingredients * | | 0.110 |
| Purified Water | Aqua | 75.58 |
| | **TOTAL** | Q.S. - 100 |

| | | |
|---|---|---|
| *Oils, proteins and extracts, such as Silk Amino acids, Hydrolyzed Rice Protein, Hydrolyzed Keratin, Aloe Barbadensis Leaf Extract, Hydrolyzed Wheat Protein, Chamomila Recutita Flower Extract, Passiflora Incarnata Seed Oil, Argania Spinosa Kernel Oil and Theobroma Grandiflorum Seed Butter PLANTAREN 2000 N UP = 50% decyl-glucoside. (4.5% final) OXITAINE CP 30 CM is 30% cocoamidopropyl betaine. (2.85% final) Cocoamidopropyl betaine to alkyl glucoside ratio = 1 : 1.58 | | |

The viscosity of the final formulation was measured using a Brookfield LVF instrument with a number 2 spindle at 6 RPM at 25°C. The viscosity was 1435 centipoise.
Formula 9

| **Trade Name** | **INCI name** | **% (w/w)** |
|---|---|---|
| Plantaren 2000 N UP | Decyl Glucoside | 9.000 |
| Mackol CAS 100N | Sodium Coco-Sulfate | 1.800 |
| Oxitaine CP 30 | Cocamidopropyl Betaine | 9.500 |
| Jaguar C 162 | Hydroxypropyl Guar Hydroxypropyltrimonium Chloride | 0.200 |
| Lamesoft PO65 | Coco-Glucoside; Glyceryl Oleate | 2.000 |
| Dye | RED 33 | 0.00004 |
| Dissolvine GL-47-S | Tetrasodium Glutamate Diacetate | 0.200 |
| PHENOXIETANOL | Phenoxyethanol | 0.360 |
| Elestab CPN | Chlorfenesin | 0.200 |
| Fragrance | Parfum | 0.300 |
| Citric Acid | Citric Acid | 0.400 |
| Additive Ingredients * | | 0.110 |
| Purified Water | Aqua | 75.930 |
| | **TOTAL** | Q.S.-100 |

| | | |
|---|---|---|
| *Oils, proteins and extracts, such as Silk Amino acids, Hydrolyzed Rice Protein, Hydrolyzed Keratin, Aloe Barbadensis Leaf Extract, Hydrolyzed Wheat Protein, Chamomila Recutita Flower Extract, Passiflora Incarnata Seed Oil, Argania Spinosa Kernel Oil and Theobroma Grandiflorum Seed Butter PLANTAREN 2000 N UP = 50% decyl-glucoside. (4.5% final) OXITAINE CP 30 CM is 30% cocoamidopropyl betaine. (2.85% final) Cocoamidopropyl betaine to alkyl glucoside ratio = 1 : 1.58 | | |

The viscosity of the final formulation was measured using a Brookfield LVF instrument with a number 2 spindle at 6 RPM at 25°C. The viscosity was 1830 centipoise.

A non-exhaustive list of aspects of the disclosure is set out in the following numbered clauses:
Clause 1. A cleansing composition comprising:
   (a) from about 3% to about 20% by weight based on the total weight of the composition of an amphoteric surfactant; and
   (b) an alkyl glucoside surfactant, wherein the ratio of alkyl glucoside surfactant to amphoteric surfactant is at least 1 to 1 and the composition is free of ethoxylated surfactants.
Clause 2. The composition according to clause 1 wherein the composition has a viscosity of at least 1000 centipoise when measured using a Brookfield LVF instrument with a number 2 spindle at 6 RPM at 25°C.
Clause 3. The composition according to clause 1 wherein the amphoteric surfactant is a betaine.
Clause 4. The composition according to clause 3 wherein the betaine is selected from the group consisting of cetyl dimethyl betaine, cocoamidopropyl betaine, and oleyl betaine.
Clause 5. The composition according to clause 4 wherein the betaine is cocoamidopropyl betaine.
Clause 6. The composition according to clause 1 wherein the alkyl glucoside surfactant is selected from the group consisting of coco glucoside and decyl glucoside.
Clause 7. The composition according to clause 6 wherein the alkyl glucoside surfactant is coco glycoside.
Clause 8. The composition according to clause 6 wherein the alkyl glucoside surfactant is decyl glycoside.
Clause 9. The composition according to clause 1 wherein the amount of alkyl glucoside surfactant ranges from about 3% to about 20% by weight based on the total weight of the composition.
Clause 10. The composition according to clause 9 wherein the amount of alkyl glucoside surfactant ranges from about 4% to about 18% by weight based on the total weight of the composition.
Clause 11. The composition according to clause 1 wherein the amount of amphoteric surfactant ranges from about 4% to about 18% by weight based on the total weight of the composition.
Clause 12. The composition according to clause 1 wherein said composition is selected from the group consisting of shampoos, 2 in 1 shampoo-conditioners, hair and body washes , washes, baths, gels, lotions, creams or combinations of two or more thereof.
Clause 13. A method of cleansing the body comprising applying a composition according to clause 12 to the body.
Clause 14. The composition according to clause 1 wherein the composition has a viscosity of at least 2000 centipoise when measured using a Brookfield LVF instrument with a number 2 spindle at 6 RPM at 25°C.
Clause 15. The composition according to clause 1 wherein the amphoteric surfactant is cocoamidopropylbetaine and the alkyl glucoside surfactant is decyl glucoside.
Clause 16. The composition according to clause 1 wherein the amphoteric surfactant is cocoamidopropylbetaine and the alkyl glucoside surfactant is coco glucoside
Clause 17. A cleansing composition comprising:
   (a) from about 3% to about 20% by weight based on the total weight of the composition of cocoamidopropylbetaine; and
   b) from about 3% to about 20% by weight based on the total weight of the composition of coco glucoside, wherein the ratio of cocoamidopropylbetaine to coco glucoside is at least 1 to 1 and the composition is free of ethoxylated surfactants and has a viscosity of at least 2000 centipoise when measured using a Brookfield LVF instrument with a number 2 spindle at 6 RPM at 25°C.

## Claims

1. A cleansing composition comprising:
(a) from about 3% to about 20% by weight based on the total weight of the composition of an amphoteric surfactant; and
(b) an alkyl glucoside surfactant, wherein the ratio of alkyl glucoside surfactant to amphoteric surfactant is at least 1 to 1 and the composition is free of ethoxylated surfactants.

2. The composition according to claim 1 wherein the composition has a viscosity of at least 1000 centipoise when measured using a Brookfield LVF instrument with a number 2 spindle at 6 RPM at 25°C.

3. The composition according to claim 1 or claim 2, wherein the amphoteric surfactant is a betaine.

4. The composition according to claim 3 wherein the betaine is selected from the group consisting of cetyl dimethyl betaine, cocoamidopropyl betaine, and oleyl betaine.

5. The composition according to claim 4 wherein the betaine is cocoamidopropyl betaine.

6. The composition according to any of the preceding claims wherein the alkyl glucoside surfactant is selected from the group consisting of coco glucoside and decyl glucoside.

7. The composition according to claim 6 wherein the alkyl glucoside surfactant is coco glycoside.

8. The composition according to claim 6 wherein the alkyl glucoside surfactant is decyl glycoside.

9. The composition according to any of the preceding claims wherein the amount of alkyl glucoside surfactant ranges from about 3% to about 20% by weight based on the total weight of the composition.

10. The composition according to claim 9 wherein the amount of alkyl glucoside surfactant ranges from about 4% to about 18% by weight based on the total weight of the composition.

11. The composition according to any of claims 1 to 8 and 10 wherein the amount of amphoteric surfactant ranges from about 4% to about 18% by weight based on the total weight of the composition.

12. The composition according to any of the preceding claims wherein the composition has a viscosity of at least 2000 centipoise when measured using a Brookfield LVF instrument with a number 2 spindle at 6 RPM at 25°C.

13. The composition according to any of claims 1 to 6 and 8 to 12 wherein the amphoteric surfactant is cocoamidopropylbetaine and the alkyl glucoside surfactant is decyl glucoside.

14. The composition according to any of claims 1 to 7 and 9 to 12 wherein the amphoteric surfactant is cocoamidopropylbetaine and the alkyl glucoside surfactant is coco glucoside.

15. The composition according to any of the preceding claims wherein said composition is selected from the group consisting of shampoos, 2 in 1 shampoo-conditioners, hair and body washes , washes, baths, gels, lotions, creams or combinations of two or more thereof.

16. A method of cleansing the body comprising applying a composition according to claim 15 to the body.

17. The composition according to claim 1, wherein:
(a) the amphoteric surfactant is cocoamidopropylbetaine;
(b) the alkyl glucoside surfactant is coco glucoside;
(c) the amount of alkyl glucoside surfactant is from about 3% to about 20% by weight based on the total weight of the composition; and
(d) the composition has a viscosity of at least 2000 centipoise when measured using a Brookfield LVF instrument with a number 2 spindle at 6 RPM at 25°C.
